# EUROPEAN PATENT SPECIFICATION

(11) **EP 3 384 881 B1**
(45) Date of publication and mention of the grant of the patent: **03.07.2019**
(21) Application number: 16870821.2
(22) Date of filing: 02.12.2016
(51) Int. Cl.: A61N 5/06, A61H 5/00

(54) **IRRADIATION DEVICE**
BESTRAHLUNGSVORRICHTUNG
DISPOSITIF D'IRRADIATION

(30) Priority: 02.12.2015 JP 2015236171
(43) Date of publication of application: 10.10.2018
(73) Proprietor: Tsubota Laboratory, Inc., Tokyo 107-0062 (JP)
(72) Inventor: TSUBOTA, Kazuo, Tokyo 107-0062 (JP); NEGISHI, Kazuno, Tokyo 107-0062 (JP); TORII, Hidemasa, Tokyo 107-0062 (JP); KURIHARA, Toshihide, Tokyo 107-0062 (JP)
(74) Representative: Mewburn Ellis LLP
(86) International application number: PCT/JP2016/085903
(87) International publication number: WO 2017/094886

(56) References cited:
- JP-A- 2006 506 111
- JP-A- 2010 506 601
- JP-A- 2014 514 038
- JP-U- 3 002 145
- US-A1- 2008 208 177
- US-A1- 2008 269 849
- US-A1- 2009 192 437
- US-A1- 2014 114 232
- US-A1- 2015 265 762
- STEPHEN B. PREPAS: 'Light, literacy and the absence of ultraviolet radiation in the development of myopia' MEDICAL HYPOTHESES vol. 70, no. 3, 04 September 2007, pages 635 - 637, XP022449003

## Description

### Field of the Invention

The present invention relates to a device that irradiates light toward an eyeball. More specifically, the present invention relates to an irradiation device that is wearable and capable of preventing myopia, slowing the progression of myopia, or the like by the action of light irradiated toward the eyeball.

### BACKGROUND ART

The number of persons with myopia continues to increase worldwide, and means for preventing the occurrence of myopia as well as means for slowing the progression of myopia are in high demand.

In Non-Patent Document 1, there is described the suppression of myopia by exposure to sunlight. Further, in Non-Patent Document 2, there is described the improvement of a biological clock by exposure to sunlight.

### PRIOR ART DOCUMENTS

### Non-Patent Documents

[Non-Patent Document 1]
   Ian Morgan, Environmental Health Perspectives, Vol. 122, No. 1, Jan., 2014.
[Non-Patent Document 2]
   Megumi Hatori, Kazuo Tsubota, Anti-Aging Medicine - Journal of Japanese Society of Anti-Aging Medicine, Vol. 11, No. 3, 065(385)-072(392).
[Non-Patent Document 3]
   Lisa A. Jones, Loraine T. Sinnott, Donald O. Mutti, Gladys L. Mitchell, Melvin L. Moeschberger, and Karla Zadnik, Investigative Ophthalmology & Visual Science, Vol. 48, No. 8, Aug., 2007.

US 2009/192437 A1 teaches a device according to the preamble of claim 1.

### SUMMARY OF THE INVENTION

### Problems to be Solved by the Invention

In recent years, sunglasses, contact lenses, and the like that prevent sunlight (ultraviolet rays and the like, in particular) from entering the eye to the extent possible have become available on the market. Further, even for buildings and vehicles, there are cases where a transmission member, such as glass, that does not transmit ultraviolet rays to the extent possible is used. Additionally, even with indoor fluorescent lamps and LEDs, products that do not emit ultraviolet rays to the extent possible are commercially available.

With the sunglasses, contact lenses, and the like described above, the purpose is to intercept light having a wavelength of 315 nm or less, which adversely affects the eye. Examples of light having a wavelength of 315 nm or less include so-called UVB (light having a wavelength of 280 to 315 nm) and UVC (light having a wavelength of 100 to 280 nm). Further, with the transmission member such as glass, the purpose is to prevent color fading of indoor furniture, sunburn, and the like.

The present inventors, in the process of researching the suppression of myopia to date, discovered that a decline in vision can be suppressed by irradiating light within a specific wavelength range among the wavelengths of a wide range included in sunlight onto the eyeball.

The present invention is the result of extensive research on which wavelength range of light is more effective in preventing myopia, suppressing the progression of myopia, and the like when irradiated onto the eyeball, on the basis of such findings. That is, an object of the present invention is to provide an irradiation device that is wearable and capable of preventing myopia, slowing the progression of myopia, or the like by the action of light irradiated toward the eyeball.

### Means for Solving the Problems

An irradiation device according to the present invention for solving the problems described above is an irradiation device comprising a light source and an instrument on which the light source is mounted. The light source emits at least light having a wavelength within a range of 350 to 400 nm, inclusive, and is disposed in a position that allows irradiation in an eyeball direction when the instrument is worn. The light source is disposed in a position 0 to 100 mm, inclusive, from a surface of the eyeball.

According to this invention, the light source is disposed in a position that allows irradiation in the eyeball direction when the instrument is worn, making it possible to appropriately irradiate the light emitted from the light source onto the eyeball. Then, the light source emits at least light having a wavelength within the range described above, and is disposed in a position 0 to 100 mm, inclusive, from the surface of the eyeball, making it possible to effectively irradiate light toward the eyeball and thus prevent myopia, slow the progression of myopia, or the like by the action of the irradiated light.

In the irradiation device according to the present invention, the light source may further emit light having a wavelength of 440 to 480 nm, inclusive, or may further comprise another light source that emits light having a wavelength of 440 to 480 nm, inclusive.

In the irradiation device according to the present invention, the light source may further emit one type or two or more types of light selectable from red light, near infrared light, and far infrared light, or may further comprise another light source that emits one type or two or more types of light selectable from red light, near infrared light, and far infrared light.

In the irradiation device according to the present invention, the light source may further emit white light, or may further comprise a light source that emits white light.

In the irradiation device according to the present invention, the light having a wavelength within the range of 350 to 400 nm, inclusive, may have an irradiance of 0.02 to 13.5 W/m², inclusive, on the eyeball surface when the instrument is worn.

In the irradiation device according to the present invention, the instrument may be eyeglasses, a cap, or an ear hook.

### Effect of the Invention

According to an irradiation device of the present invention, it is possible to provide an irradiation device that is wearable and capable of preventing myopia, slowing the progression of myopia, or the like by the action of light irradiated toward an eyeball.

### BRIEF DESCRIPTION OF THE DRAWINGS

Fig. 1 is a schematic view illustrating an example (an eyeglasses type) of an irradiation device according to the present invention.
Fig. 2 is a schematic view illustrating an example (a cap type) of the irradiation device according to the present invention.
Fig. 3 is a schematic view illustrating an example (an ear hook type) of the irradiation device according to the present invention.
Fig. 4 is a schematic view illustrating an example (an eyeball mounting type) of the irradiation device according to the present invention.
Fig. 5 is a graph showing the relationship between wavelength and spectral irradiance of light of a violet fluorescent lamp used in Experiment 1.
Fig. 6 is a graph showing a change in axial lengths of chicks irradiated with violet light and chicks not irradiated with violet light in Experiment 1.
Figs. 7A to 7C are examples of an eyeglasses-type irradiation device used in Experiment 3, Fig. 7A being an example with an LED provided on an outer end side, Fig. 7B being an example with an LED provided in the middle, and Fig. 7C being an example with an LED provided on an inner end side.
Fig. 8 is a light spectrum of a light source used in Experiment 3.
Fig. 9 is a schematic diagram illustrating an example of an irradiation device comprising a light-emitting element of light having a wavelength within a range of 350 to 400 nm, inclusive, and a light-emitting element of white light.
Fig. 10 is a schematic diagram illustrating an example of an irradiation device comprising a light-emitting element of light having a wavelength within a range of 350 to 400 nm, inclusive, and a light-emitting element of white light.

### Embodiments of the Invention

An irradiation device according to the present invention is described below with reference to the drawings. The present invention is not limited to the embodiments and examples below, and various modifications can be made as long as the gist of the present application is included.

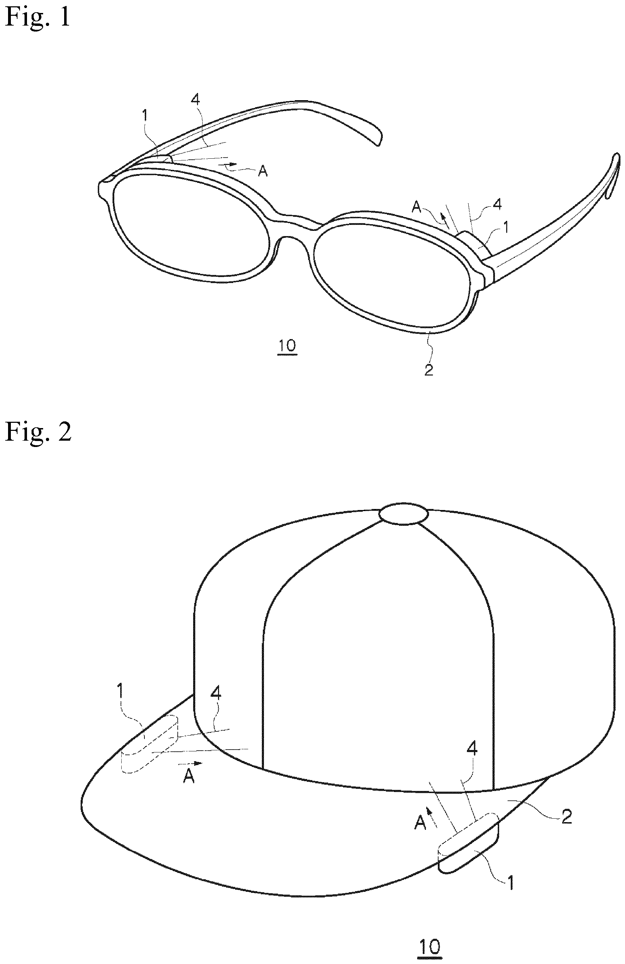

An irradiation device 10 according to the present invention comprises a light source 1, and an instrument 2 on which the light source 1 is mounted, as illustrated in Figs. 1 to 4. The light source 1 emits at least a light 4 having a wavelength within a range of 350 to 400 nm, inclusive, and is disposed in a position that allows irradiation in an eyeball direction A when the instrument 2 is worn. Further, the light source 1 is preferably disposed in a position 0 to 100 mm, inclusive, from a surface of the eyeball.

With the light source 1 disposed in a position that allows irradiation in the eyeball direction A when the instrument 2 is worn, such an irradiation device 10 is capable of appropriately irradiating the light 4 emitted from the light source 1 onto an eyeball 3. Then, the light source 1 emits at least the light 4 having a wavelength within the range described above, and is disposed in a position 0 to 100 mm, inclusive, from the surface of the eyeball, making it possible to accurately and effectively irradiate light toward the eyeball and thus prevent myopia, slow the progression of myopia, or the like by the action of the irradiated light.

The components of the irradiation device according to the present invention are described in detail below. It should be noted that, in the present application, the term "light" is used in the sense of electromagnetic waves, and has the same meaning when replaced with the term "electromagnetic waves."

### (Instrument)

The instrument 2, as illustrated in Figs. 1 to 4, is for mounting the light source 1. With the light source 1 mounted on this instrument 2, the light 4 emitted from the light source 1 is toward the eyeball 3. Examples of the instrument 2 preferably include eyeglass frames such as illustrated in Fig. 1, but are not limited thereto.

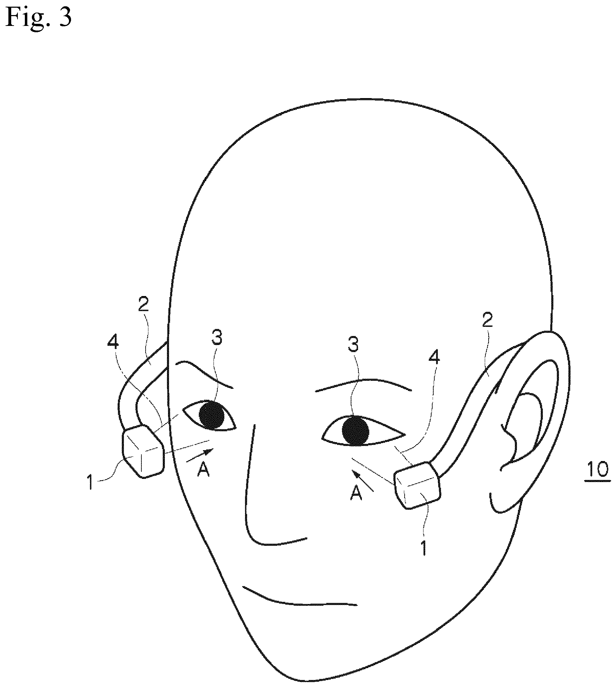

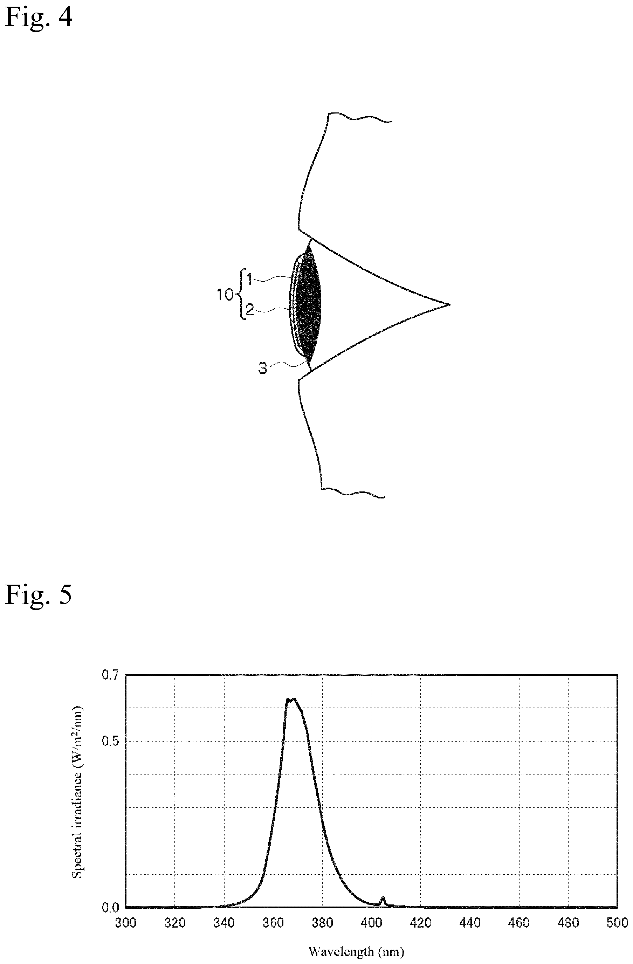

Other than the eyeglass frames, a cap-type instrument such as illustrated in Fig. 2, an ear hook-type instrument such as illustrated in Fig. 3, or a contact-type instrument directly worn on the eyeball 3 such as illustrated in Fig. 4 may be the instrument 2. In each case, the instrument 2 is worn on a face, a head, an ear, or the like near the eyes, allowing the light 4 emitted from the light source 1 to point in a direction A of the eyeball 3. In such an instrument, the instrument 2 may be other than the one illustrated in Figs. 1 to 4.

With such a form, when a person wears the instrument 2, the light source 1 can be disposed in a position allowing irradiation in the eyeball direction A. As a result, the light 4 emitted from the light source 1 can be appropriately irradiated onto the eyeball 3.

According to the present invention, the light source 1 mounted on the instrument 2 is preferably disposed in a position 0 to 100 mm, inclusive, from the surface of the eyeball 3. "0 mm from the surface of the eyeball 3" indicates, for example, a case of a contact-type wearable instrument that allows the light source to be directly worn on the eyeball 3. With the light source 1 disposed in such a position, the light can be accurately and effectively irradiated toward the eyeball, making it possible to prevent myopia or slow the progression of myopia by the action of the irradiated light. It should be noted that "to 100 mm, inclusive, from the surface of the eyeball 3" takes into consideration separation of the light source 1 from the face or the head when disposed in a position exceeding 100 mm, which causes a decrease in the accuracy of irradiation with respect to the eyeball, possible obstruction, and a decrease in the number of options available as the light source 1.

### (Light source)

The light source 1 is disposed in a position allowing irradiation of the light 4 in the eyeball direction A when a person wears the instrument 2. The position where the light source 1 is disposed differs according to the form of the instrument 2. In the eyeglasses-type instrument in Fig. 1, for example, the light source 1 may be provided to frames. While the position where the light source 1 is provided to the frames is not particularly limited, the light source 1 may be provided around a whole circumference of the frames, or in one or two or more positions in any of four directions of a frame upper side (top rim side), a frame outer end side (temple side), a frame inner end side (bridge side), and a frame lower side (under rim side). In the cap-type instrument in Fig. 2, the light source 1 may be provided on a brim or the like of the cap. In the ear hook-type instrument in Fig. 3, the light source 1 may be provided to an arm portion extending from the ear. In the contact-type instrument on the eyeball in Fig. 4, the light source 1 may be integrated with the instrument.

The light 4 emitted from the light source 1 is a light having a wavelength within a range of 350 to 400 nm, inclusive. With the light 4 having a wavelength within this range irradiated onto the eyeball 3, it is possible to prevent myopia, slow the progress of myopia, or the like. A "light having a wavelength within the range of 350 to 400 nm, inclusive" may be any light having a wavelength within the range of 350 to 400 nm, inclusive. For example, the light may be 350 to 380 nm. For example, the light may be 360 to 390 nm. For example, the light may be 370 to 400 nm. Naturally, the light may be 350 to 400 nm.

Further, such a light 4 needs only to include at least light having a wavelength within the range of 350 to 400 nm, inclusive, thereby preventing (suppressing the occurrence of) myopia or suppressing the progression of myopia. For example, the light may have a light spectrum having a peak within a wavelength range of 350 to 400 nm, inclusive. For example, the light may have a light spectrum not having a peak within a wavelength range of 350 to 400 nm, inclusive. It should be noted that light "having a peak within a wavelength range of 350 to 400 nm, inclusive" may have a light spectrum having a peak at a wavelength of 365 nm, or a light spectrum having a peak at 380 nm, for example. Further, "not having a peak within a wavelength range of 350 to 400 nm, inclusive" indicates a case where light of a light spectrum having a peak at a wavelength exceeding 400 nm is emitted even within the range of 350 to 400 nm, inclusive, for example. It should be noted that light of a light spectrum having a peak at a wavelength less than 350 nm may include light of 315 nm or less, which adversely affects the eye, and therefore is preferably excluded according to the present invention.

It should be noted that the light 4 may be only light having a wavelength within the range of 350 to 400 nm, inclusive. In this case, light beyond the wavelength range of 350 to 400 nm, inclusive (light having a wavelength less than 350 nm or exceeding 400 nm) may be slightly included. Examples of "slightly" include a mode included as noise much smaller than that of the irradiance described later, and a mode minutely included as a broad base portion of the light spectrum. While the extent of such light is not particularly limited, the light is in an amount that does not contribute to the prevention (suppression of occurrence) of myopia or the suppression of progression of myopia, and thus can be set to less than about 10% of the amount of light within the range of 350 to 400 nm, inclusive.

In Non-Patent Document 3, outdoor activity of 14 hours or more per week is stated to significantly decrease the probability of a myopia outbreak. Nevertheless, the type of outdoor activity that reduces the probability of a myopia outbreak is not clearly defined. For example, whether incidental movement of the eyeball, such as looking at a landscape, plants, animals, or the like simply by being outdoors, physical movement, or stress reduction as a result of being outdoors is effective, or whether a factor (intensity of visible rays, intensity of ultraviolet rays, exposure time, or the like) of an environment that allows exposure to natural light is effective has not been made clear in prior art. In particular, the wavelength range of light effective in preventing myopia has not been specified.

According to the present invention, when the instrument 2 is worn in a predetermined position of 100 mm or less from the surface of the eyeball 3, the irradiance on the surface of the eyeball 3 is preferably 0.02 W/m² or greater as an integral value within the wavelength range of 350 to 400 nm, inclusive. A more preferable irradiance is 0.25 W/m² or greater and, more preferably, 0.5 W/m² or greater. With the irradiation of the light 4 having such an irradiance onto the surface of the eye, it is possible to prevent myopia or slow the progression of myopia by the action of the light 4. In Experiment 1 described later, light exposure is performed for 2 hours or longer per day at an irradiance of 0.25 W/m² or greater. This way, the dosage of light received by the eye per day is 1,800 J/m² or greater. It should be noted that, as long as the irradiation time per day or the number of days of irradiation is increased, the irradiance may be less than 0.25 W/m², allowing an irradiance of 0.02 W/m² or greater as described above.

The irradiance of the light was calculated by measuring the spectral irradiance of sunlight and using the irradiance of sunlight as a reference. For example, the light intensity (irradiance) of sunlight having a wavelength of 350 to 400 nm is 33.3 W/m² according to calculations based on international standard data (AM1.5G). Further, as an actual measurement value, for example, the light intensity (irradiance) of sunlight having a wavelength of 350 to 400 nm at Keio University Shinanomachi Campus (35 Shinanomachi, Shinjuku-ku, Tokyo, Japan) at noon (12:00 PM) on May 26, 2015 was 1.6 W/m² in the horizontal direction facing north. Thus, in the present invention, an intensity of approximately 15% or greater (preferably approximately 30% or greater) of 1.6 W/m² in the horizontal direction facing north is determined as preferred, and therefore the irradiance of the light 4 emitted from the light source 1 is preferably 0.25 W/m² or greater (preferably 0.5 W/m² or greater) on the surface of the eyeball 3 when the instrument 2 is worn. It should be noted that, similar to the above, as long as the irradiation time per day is increased, the irradiance may be less than 0.25 W/m², allowing an irradiance of 0.02 W/m² or greater as described above.

As the light source 1 having such an irradiance, LEDs and the like available on the market may be selected and used. For example, a bullet-shaped light (LED "NSPU510CS" manufactured by Nichia Corporation, peak wavelength: 375 nm, for example) or the like may be used. Further, the irradiance may be controlled using a filter or the like capable of controlling light transmittance. The spectral irradiance can be measured by a spectrometer, or may be calculated by integrating an integral value (irradiance) in a wavelength range in which the spectral irradiance is to be found. The spectral irradiance is measured as a value on the surface of the eyeball 3 when the instrument 2 is mounted. Thus, with the light source 1 mounted on the instrument 2 being disposed in a position 0 to 100 mm, inclusive, from the surface of the eyeball 3 as described above, evaluation is conducted at a position where the light 4 from the light source 1 disposed within that range reaches the position of the eyeball 3.

### (Light of other wavelength ranges)

The light 4 emitted from the light source 1 is acceptable as long as the wavelength is at least 350 to 400 nm, inclusive, and thus may be only light having a wavelength of 350 to 400 nm, inclusive, or may include light having another wavelength range. When the light is only light having a wavelength of 350 to 400 nm, inclusive, an LED that emits only light in that range may be used, or a filter (light absorbing filter) that does not allow transmission of light other than light having a wavelength of 350 to 400 nm, inclusive, from the light that includes light of other wavelength ranges may be used. Further, light of another wavelength range that exceeds 400 nm may be included.

For example, the light source 1 may further emit light having a wavelength of 440 to 480 nm, inclusive, or may further comprise another light source that emits light having a wavelength of 440 to 480 nm, inclusive. This makes it possible to contribute to an effect of improving a biological clock as well.

Further, for example, the light source 1 may further emit one type or two or more types of light selectable from red light, near infrared light, and far infrared light, or may further comprise another light source that emits one type or two or more types of light selectable from red light, near infrared light, and far infrared light. While there are various definitions of the infrared region, here red light refers to light having a wavelength of approximately 600 to 780 nm, near infrared light refers to light having a wavelength of approximately 780 to 3,000-4,000 nm, and far infrared light refers to light having a wavelength from that value to 1,000 µm, inclusive, as generally defined in the heating field. It should be noted that, in the chemical analysis field, the concepts of mid-infrared light and ultra-far infrared light also apply, and the red light refers to light having a wavelength of approximately 740 to 780 nm, near infrared light refers to light having a wavelength of approximately 780 to 1,500 nm, mid-infrared light refers to light having a wavelength of approximately 1,500 to 5,600 nm, far infrared light refers to light having a wavelength of approximately 5,600 to 25,000 nm, and ultra-far infrared light refers to light having a wavelength of approximately 25,000 nm to 1,000 µm, inclusive.

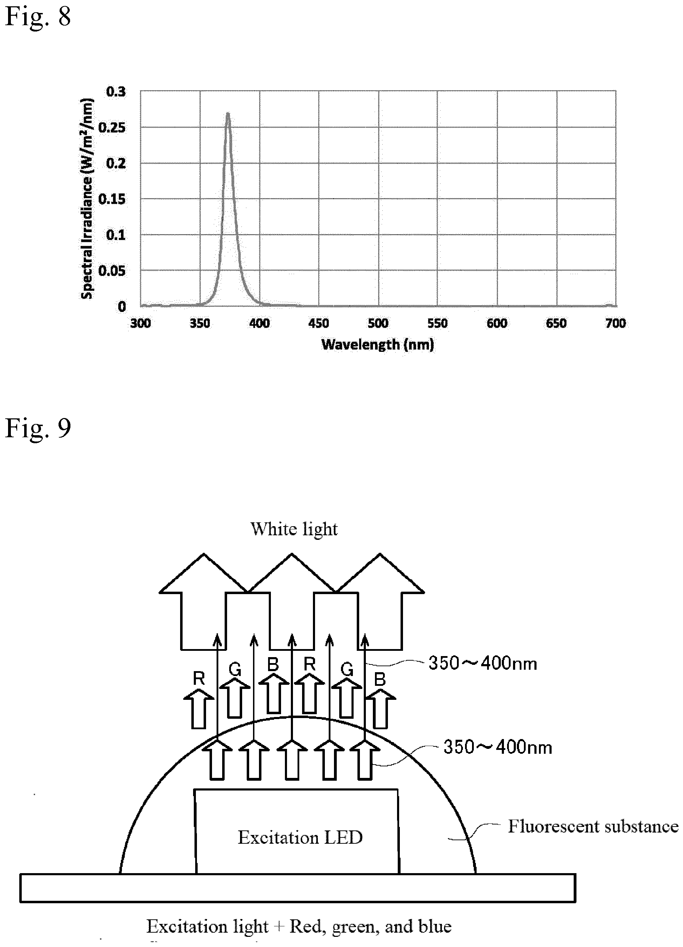

Further, for example, the light source 1 may further emit white light, or may further comprise another light source that emits white light. Examples include the light-emitting element illustrated in Fig. 9. The light-emitting element illustrated in Fig. 9 comprises a light-emitting element that emits excitation light having a wavelength within the range of 350 to 400 nm, inclusive, and a light-emitting element that emits white light. This light-emitting element is an example of an element that includes an excitation light-emitting portion (LED) that emits excitation light having a wavelength within the range of 350 to 400 nm, inclusive, and fluorescent substances of red, green, and blue provided so as to cover the excitation light-emitting portion. With such a configuration, when the excitation light having a wavelength within the range of 350 to 400 nm, inclusive, is irradiated onto the fluorescent substances, white light formed by the three colors red, green, and blue is emitted. Apart of the light (excitation light) having a wavelength within the range of 350 to 400 nm, inclusive, is transmitted through the fluorescent substances as illustrated. Such a light-emitting element may be used as a white light source that includes light for producing a myopia preventive effect.

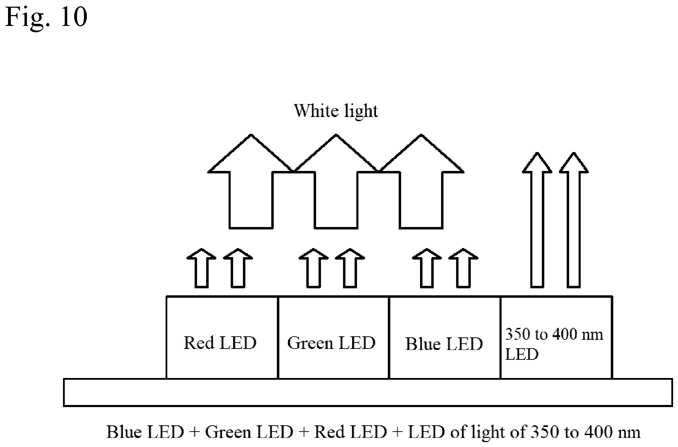

Further, other examples include the light-emitting element illustrated in Fig. 10. The light-emitting element illustrated in Fig. 10 comprises a light-emitting element that emits light having a wavelength within the range of 350 to 400 nm, inclusive, and an RGB light-emitting element that emits white light. This light-emitting element is an example of an element that includes a light-emitting portion that emits light having a wavelength within the range of 350 to 400 nm, inclusive, and the light-emitting portions (LEDs) of R (red), G (green), and B (blue). Such a configuration may be used as a white light source that includes light for producing a myopia preventive effect.

It should be noted that, with a general RGB light-emitting element, light having a wavelength within the range of 350 to 400 nm, inclusive, is substantially not included in the white light, even when the element can produce pseudo white light close to sunlight. Nevertheless, the light-emitting elements illustrated in Figs. 9 and 10 emit light having a wavelength within the range of 350 to 400 nm, inclusive, and this light is white light (natural light) that has a wider spectral width overall, and is closer to sunlight. Thus, the light-emitting elements illustrated in Figs. 9 and 10 are capable of producing the exceptional effect of selectively emitting light having a wavelength within the range of 350 to 400 nm, inclusive, which particularly has a myopia preventive effect. The white light may be emitted by means shown in Figs. 9 and 10, or may be emitted by other means. Examples of other means include a light-emitting element formed by R (red) and G (green) (B: not included) obtained by controlling the emission wavelength, in addition to the excitation light having a wavelength within the range of 350 to 400 nm, inclusive.

### (Irradiation device)

The irradiation device 10 comprises the light source 1 and the instrument 2 on which the light source 1 is mounted, as illustrated in Figs. 1 to 4, and the light source 1 may be attached to the instrument 2 by adhesive or mechanically by a screw or a fastener. Further, a power source (not illustrated) that supplies power to the light source 1 may be a battery embedded or mounted in the instrument 2, or may be drawn by a cable to a battery mounted in another position. Additionally, when stationary in one location, the irradiation device 10 may be connected to a residential power source or the like.

Furthermore, a controller or timer function of the light source 1 may be provided. Examples of controller functions include functions for varying the irradiance or adjusting the irradiation angle toward the eyeball. Further, examples of timer functions include a function that allows the light irradiance time to be set. Such controller and timer functions may be provided in an integrated manner with the instrument 2 or may be provided separately.

The irradiation device 10 comprising the light source 1 described above emits at least the light 4 having a wavelength within the range of 350 to 400 nm, inclusive, and is disposed in a position that allows irradiation in the eyeball direction when the instrument 2 is worn. Further, the light source 1 is disposed in a position 0 to 100 mm, inclusive, from the surface of the eyeball. As a result, it is possible to prevent myopia or slow the progression of myopia by the action of the light 4 accurately irradiated toward the eyeball 3. Further, a predetermined amount of light is irradiated onto the eyeball at a specific wavelength, resulting in the advantage of achieving the effect of preventing myopia (light having a wavelength of 350 to 400 nm, inclusive) and improving the biological clock (light having a wavelength of 440 to 480 nm, inclusive).

### Examples

The present invention is described in further detail below using examples.

### [Experiment 1]

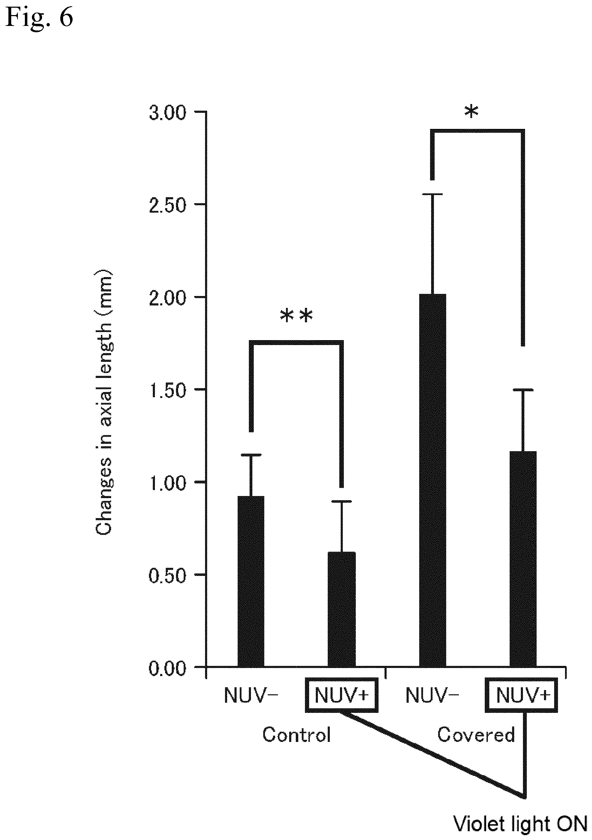

A light irradiation experiment on chicks was conducted. The light sources used included a violet fluorescent lamp having a peak wavelength of 365 nm, and a regular white fluorescent lamp (that does not emit light of 400 nm or less). Fig. 5 is a graph showing the relationship between wavelength and the spectral irradiance of the light of the violet fluorescent lamp used in Experiment 1. A fiber multichannel spectrometer (Blue Wave manufactured by StellarNet Inc. (US)) was used for measurements. A chick is known to develop myopia in an eye (shielded eye) that is covered with a transparent hemisphere (refer to Seko, et al., Invest. Ophthalmol. Vis. Sci, May, 1995, Vol. 36, No. 6, pp. 1183-1187, for example). Here, an experiment was conducted with White-Leghorn chicks by covering one eye of each chick with a transparent hemisphere six days after birth, and separating the chicks into a non-irradiation group of 15 chicks not irradiated with violet light, and an irradiation group of 15 chicks irradiated with violet light. The average irradiance in a horizontal direction at the height of the eye of the chick was set to 4.12 W/m², a 12-hour light-dark cycle was continued each day for seven days, and the degree of development of myopia in the shielded eye was surveyed. With each group, white fluorescent light was irradiated. The eye not covered with the transparent hemisphere is called a control eye. Fig. 6 is a graph showing a change in axial lengths of the chicks irradiated with violet light and the chicks not irradiated with violet light in Experiment 1.

The axial lengths of the shielded eyes of the non-irradiation group were measured on the 13th day (7 days after the start of shielding). The axial length of the eye was measured using US-4000 (manufactured by Nidek Co., Ltd.) in mode B. As a result, the change in the average axial length of the shielded eyes of the irradiation group was significantly smaller than that in the average axial length of the shielded eyes of the non-irradiation group, and the degree of progression of myopia of the irradiation group was smaller than that of the non-irradiation group. In the control group as well, the same tendencies were confirmed.

### [Experiment 2]

The eyeglasses-type irradiation device illustrated in Fig. 1 was manufactured. A button-type light (the LED "NSSU123T" manufactured by Nichia Corporation, measured value of peak wavelength: 373 nm) was mounted onto the frames of the eyeglasses type, and the irradiance was electrically set to 0.25 W/m². Thus, the irradiation device according to the present invention was manufactured. It should be noted that the supply of power to the light source used was a residential AC power supply.

### [Experiment 3]

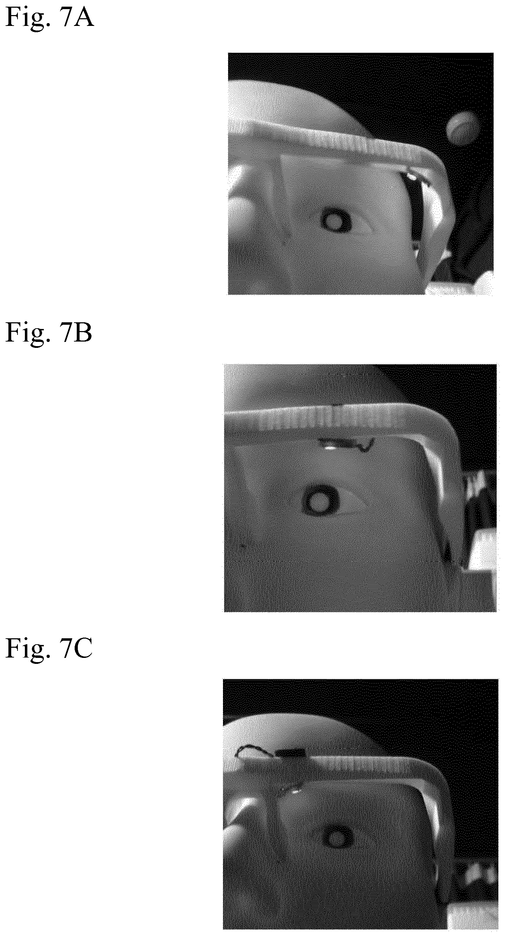

The eyeglasses-type irradiation device shown in Figs. 7A to 7C was manufactured. For the light source, an LED ("NSSU123T," measured value of peak wavelength: 373 nm) manufactured by Nichia Corporation was mounted to frames without lenses. Fig. 7A is an example of an LED provided on a temple side (frame outer end side) of the top rim (frame upper side), Fig. 7B is an example of an LED provided in a middle position of the top rim (frame upper side), and Fig. 7C is an example of an LED provided on a bridge side (frame inner end side) of the top rim (frame upper side). Fig. 8 is a light spectrum of the light source measured using a fiber multichannel spectrometer (Blue Wave manufactured by StellarNet Inc. (US)).

This eyeglasses-type irradiation device was mounted on a mannequin, and the irradiance on the eyeball surface was measured. The irradiance was measured with a probe of the spectrometer described above installed on the eyeball surface. A distance from the light source (refer to Figs. 7A to 7C) provided on the frame to the eyeball was approximately 3 cm in the case of Fig. 7A, approximately 2 cm in the case of Fig. 7B, and approximately 2.5 cm in the case of Fig. 7C. The voltages applied to the LED and the measurement results of the irradiance at the time are shown in Table 1. Based on the results in Table 1, the eyeglasses-type irradiation device capable of varying the irradiance within the range of 0.02 W/m² to 13.5 W/m² was manufactured. This irradiation device was used, making it possible to set a preferred irradiance.

**[Table 1]**

| Set Voltage (V) | Outer End Installation Irradiance (W/m²) | Middle Installation Irradiance (W/m²) | Inner End Installation Irradiance (W/m²) |
|---|---|---|---|
| 5.0 | 3.14 | 13.50 | 7.60 |
| 4.5 | 2.34 | 9.80 | 5.50 |
| 4.0 | 1.48 | 6.40 | 3.60 |
| 3.5 | 0.67 | 2.80 | 1.55 |
| 3.3 | 0.36 | 1.51 | 0.85 |
| 3.2 | 0.23 | 0.77 | 0.52 |
| 3.1 | 0.09 | 0.35 | 0.24 |
| 3.0 | 0.02 | 0.08 | 0.05 |

### Descriptions of Reference Numerals

- 1: Light source
- 2: Instrument
- 3: Eyeball
- 4: Light
- 10: Irradiation device
- A: Eyeball direction

## Claims

1. An irradiation device comprising:
a light source; and
an instrument for mounting the light source;
the light source emitting at least light having a wavelength within a range of 350 to 400 nm, inclusive, and being disposed in a position allowing irradiation in an eyeball direction when the instrument is mounted; and
the light source being disposed in a position 0 to 100 mm, inclusive, from a surface of the eyeball, **characterized in that**
the light having a wavelength within the range of 350 to 400 nm, inclusive, has an irradiance of 0.02 to 13.5 W/m², inclusive, on an eyeball surface when the instrument is worn.

2. The irradiation device according to claim 1, wherein
the light source further emits light having a wavelength of 440 to 480 nm, inclusive, or further comprises another light source that emits light having a wavelength of 440 to 480 nm, inclusive.

3. The irradiation device according to claim 1, wherein
the light source further emits one type or two or more types of light selectable from red light, near infrared light, and far infrared light, or further comprises another light source that emits one type or two or more types of light selectable from red light, near infrared light, and far infrared light.

4. The irradiation device according to claim 1, wherein
the light source further emits white light, or further comprises a light source that emits white light.

5. The irradiation device according to any one of claims 1 to 4, wherein
the instrument is an eyeglasses type, a cap type, or an ear hook type.

## Patentansprüche

1. Bestrahlungsvorrichtung, umfassend:
eine Lichtquelle; und
ein Instrument zum Anbringen der Lichtquelle;
wobei die Lichtquelle zumindest Licht, das eine Wellenlänge in einem Bereich einschließlich von 350 bis 400 nm aufweist, emittiert und in einer Position angeordnet ist, die eine Bestrahlung in eine Richtung des Augapfels ermöglicht, wenn das Instrument angebracht ist; und
wobei die Lichtquelle in einer Position einschließlich 0 bis 100 mm von einer Oberfläche des Augapfels angeordnet ist,
**dadurch gekennzeichnet,**
**dass** das Licht, das eine Wellenlänge im Bereich einschließlich von 350 bis 400 nm aufweist, eine Bestrahlungsstärke einschließlich von 0,02 bis 13,5 W/m² auf einer Augapfeloberfläche aufweist, wenn das Instrument getragen wird.

2. Bestrahlungsvorrichtung gemäß Anspruch 1,
wobei die Lichtquelle ferner Licht emittiert, das eine Wellenlänge einschließlich von 440 bis 480 nm aufweist, oder ferner eine weitere Lichtquelle umfasst, die Licht emittiert, das eine Wellenlänge einschließlich von 440 bis 480 nm aufweist.

3. Bestrahlungsvorrichtung gemäß Anspruch 1,
wobei die Lichtquelle ferner eine Art oder zwei oder mehrere Arten von Licht emittiert, das aus rotem Licht, nahem Infrarotlicht und fernem Infrarotlicht auswählbar ist, oder ferner eine weitere Lichtquelle umfasst, die eine Art oder zwei oder mehrere Arten von Licht umfasst, das aus rotem Licht, nahem Infrarotlicht und fernem Infrarotlicht auswählbar ist.

4. Bestrahlungsvorrichtung gemäß Anspruch 1,
wobei die Lichtquelle ferner weißes Licht emittiert oder ferner eine Lichtquelle umfasst, die weißes Licht emittiert.

5. Bestrahlungsvorrichtung gemäß einem der Ansprüche 1 bis 4,
wobei das Instrument eine Art von Brille, eine Art von Kappe oder eine Art von Ohrbügel ist.

## Revendications

1. Dispositif d'irradiation comprenant :
une source de lumière ; et
un instrument destiné à monter la source de lumière ;
la source de lumière émettant au moins une lumière ayant une longueur d'onde comprise entre 350 et 400 nm compris, et étant disposée à un emplacement qui permet une irradiation dans une direction de globe oculaire lorsque l'instrument est monté ; et
la source de lumière étant disposée à un emplacement situé à 0 à 100 mm, compris, d'une surface du globe oculaire, **caractérisé en ce que**
la lumière ayant une longueur d'onde comprise entre 350 et 400 nm, compris présente une irradiance de 0,02 à 13,5 W/m² comprise, sur une surface de globe oculaire lorsque l'instrument est porté.

2. Dispositif d'irradiation selon la revendication 1, dans lequel
la source de lumière émet en outre une lumière ayant une longueur d'onde de 440 à 480 nm compris, ou comprend en outre une autre source de lumière ayant une longueur d'onde de 440 à 480 nm compris.

3. Dispositif d'irradiation selon la revendication 1, dans lequel
la source de lumière émet en outre un type ou deux types ou plus de lumière qui peut être choisie entre une lumière rouge, une lumière de proche infrarouge, et une lumière d'infrarouge lointain, ou comprend en outre une autre source de lumière qui émet un type ou deux types ou plus de lumière qui peut être choisie parmi une lumière rouge, une lumière de proche infrarouge, et une lumière d'infrarouge lointain.

4. Dispositif d'irradiation selon la revendication 1, dans lequel
la source de lumière émet en outre une lumière blanche, ou comprend en outre une source de lumière qui émet une lumière blanche.

5. Dispositif d'irradiation selon l'une quelconque des revendications 1 à 4, dans lequel
l'instrument est de type globe oculaire, casquette, ou oreillette.
